# EUROPEAN PATENT APPLICATION

(11) **EP 4 591 854 A1**
(43) Date of publication of application: **30.07.2025**
(21) Application number: 24153321.5
(22) Date of filing: 23.01.2024
(51) Int. Cl.: A61K 9/06, A61K 38/16, A61K 47/02, A61P 25/32

(54) **ALCOHOL DETOXIFICATION**

(71) Applicant: ETH Zurich, 8092 Zürich (CH); Universiteit Gent, 9000 Gent (BE)
(72) Inventor: Su, Jiaqi, 8006 Zürich (CH); Mezzenga, Raffaele, 8604 Volketswil (CH)
(74) Representative: E. Blum & Co. AG

(57) **Abstract**

The present invention relates to the field alcohol intoxication and its treatment and discloses novel hybrid materials and pharmaceutical compositions, their manufacturing, and their use in the context of excessive alcohol consumption. The hybrid materials disclosed comprise (a) amyloid proteins, such as fibrous amyloid proteins, and (b) a multitude of single-site iron coordinated to said amyloid proteins. These hybrid materials may be converted to hydrogels, pharmaceutical compositions comprising such hydrogels, and oral dosage forms comprising such hydrogels. Further disclosed are methods for manufacturing such hybrid material and hydrogels as well as methods of using such hybrid materials and hydrogels in the treatment of alcoholism.

## Description

The present invention relates to the field alcohol intoxication and its treatment, including both, detoxification and prophylaxis. Accordingly, the invention discloses novel materials and compositions, their manufacturing, and their use in the context of excessive alcohol consumption.

### Background

Providing strategies to reduce global health impacts induced by alcohol prevalence is both a provocative and challenging topic. Despite the positive effects observed with intravenous applications of natural enzyme complexes, their insufficient activities and complicated usage often result in the accumulation of toxic acetaldehyde, which raises important clinical concerns, highlighting the pressing need for stable oral strategies.

While various therapies exist to mitigate the effects of alcohol, they mainly constitute supportive measures, relying on endogenous enzymes to metabolize alcohol within the body. These substances that function as alcohol antidotes only temporarily relieve alcoholics from symptoms, such as nausea and headaches, and fail to address other vital issues, such as drowsiness, exhaustion, and chronic alcoholism. In recent years, nanocomplexes composed of multiple complementary hepatic enzymes have emerged as a promising approach for accelerating human alcohol metabolism. Although promising, a significant obstacle arises from the insufficient activity of commercially available aldehyde dehydrogenase (ALDH) or aldehyde oxidase (ADOx) enzymes, leading to the accumulation of a more hazardous intermediate, acetaldehyde, and further damage to human organs.

For example, Xie et al (Journal of Ethnopharmacology 282 (2022): 114593) discuss the protective effects and mechanisms of modified Lvdou Gancao (MLG) decoction on acute alcohol intoxication in mice.

Further, Xu et al (Advanced Materials 30.22 (2018): 1707443) evaluate a hepatocyte-mimicking antidote for alcohol intoxication through the codelivery of the nanocapsules of alcohol oxidase (AOx), catalase (CAT), and aldehyde dehydrogenase (ALDH) to the liver, where AOx and CAT catalyze the oxidation of alcohol to acetaldehyde, while ALDH catalyzes the oxidation of acetaldehyde to acetate.

Still further, Chen et al (Alcoholism: Clinical and Experimental Research 38.7 (2014): 1839-1846) assess the effect of Flos Puerariae extract (FPE) on alcohol metabolism, hepatic injury, and memory impairment following acute ethanol (EtOH) intoxication in mice.

Still further, Liu et al (Nature nanotechnology 8.3 (2013): 187-192) show that two or more enzymes with complementary functions can be assembled and encapsulated within a thin polymer shell to form enzyme nanocomplexes. These nanocomplexes exhibit improved catalytic efficiency and enhanced stability when compared with free enzymes. These nanocomplexes are considered useful as antidotes and preventive measures for alcohol intoxication.

While the prior art discussed above is considered a promising approach in the treatment of alcoholism, it requires non-oral administration of the compositions disclosed. It would be beneficial to provide a safe and efficient oral administration route for treatment of alcoholism.

Nanozymes have been proposed as possible alcohol detoxification strategies. For example, Geng et al. (ACS Nano 2023, 17, 8, 7443-7455) relied on mesoporous metal organic framework nanozyme combined with enzymes for alcohol detoxification in mice models. Graphene oxide quantum dots have also been proposed as a nanozyme with peroxidase-like activity for alcohol intoxication, Sun et al. (ACS Appl. Mater. Interfaces 2017, 9, 14, 12241-12252). However, the safety issues associated with these materials are such to make a practical application in humans still far.

In the context of nutritional materials, Bolisetty et al (WO2018/166947) disclose composite materials comprising amyloid fibrils and nanoparticulate iron minerals (such as oxides, hydroxides and salts of iron). This document shows improved bioavailability of iron as a food supplement and confirms fast enzymatic digestion of the composite materials disclosed therein; it is silent, however, on the treatment of alcoholism.

### Summary of the Invention

The present invention will be described in more detail below, referring to the first, second and third aspect of the invention. The **first aspect** is directed to new materials (hybrid materials and hydrogels), the **second aspect** is directed to pharmaceutical compositions and their uses while the **third aspect** is directed to the manufacturing of the above new materials and pharmaceutical compositions. It is understood that the various embodiments, preferences and ranges as provided / disclosed in this specification may be combined at will. Further, depending of the specific embodiment, selected definitions, embodiments or ranges may not apply.

Unless otherwise stated, the following **definitions** shall apply in this specification:
As used herein, the term "a," "an," "the" and similar terms used in the context of the present invention (especially in the context of the claims) are to be construed to cover both the singular and plural unless otherwise indicated herein or clearly contradicted by the context. Further, as used herein, the terms "including", "containing" and "comprising" are used herein in their open, non-limiting sense. The term "containing" shall include "comprising", "essentially consisting of' and "consisting of".

**Pharmaceutical Compositions:** The term pharmaceutical composition in known in the field and describes any composition suitable for the treatment, prevention or delay of progression of a disease in a subject in need thereof. The term particularly includes formulations adapted for oral administration, e.g. in liquid or solid dosage form. Suitable liquid dosage forms include oral solutions / suspensions, such as a hydrogel dissolved in a pharmaceutically acceptable diluent. Suitable solid dosage forms include capsules, such as an encapsulated hydrogel.

Pharmaceutical doses for the minerals discussed may vary; the skilled person is in a position to prepare pharmaceutical compositions meeting with these criteria. For example, an inventive hydrogel may be administered in a range of 1 mL kg-1 to 30 mL kg-1 body weight (BW), such as 10 mL kg-1 BW.

**Alcoholism:** The term is known in the field and includes alcohol abuse, alcohol dependence, and alcohol use disorder. The term shall include acute alcohol intoxication and chronic alcohol intoxication of a subject, specifically a human being. The materials and methods described herein are specifically designed to alleviate the negative impacts of ethanol consumption on the body.

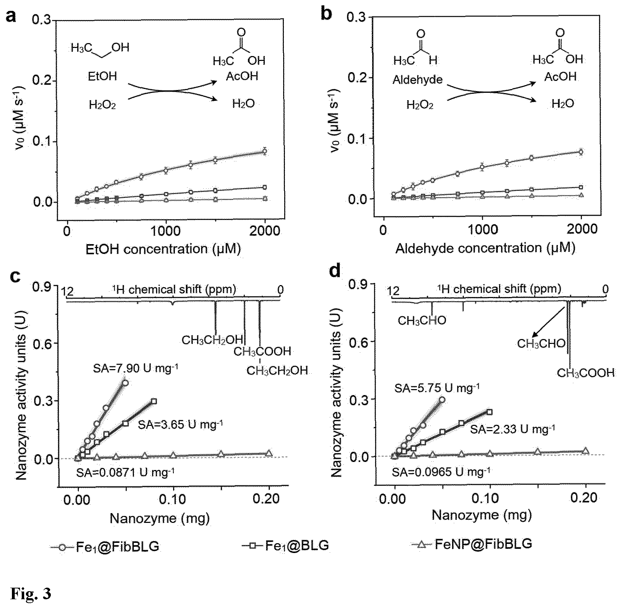

The present invention will be better understood by reference to the **figures.**
Fig.1 illustrates the synthesis process of Fe1@FibBLG.
Fig.2 shows analytical details for the inventive hybrid material.
   a. Normalized XANES spectra at Fe K-edge of Fe1@FibBLG along with reference samples. [x-axis Photo energy (eV), y-axis XANES (a.u.)]
   b. Fitting curves of the EXAFS of Fe1@FibBLG in the r-space and K-space (inset). [x-axis R (Angstroem), y-axis FT (a.u.)]
Fig.3 compares the inventive hybrid material possessing a multitude of single-site iron, namely Fe1@FibBLG [circles], and Fe1@BLG [squares] with hybrid material possessing a multitude of nanoparticulate iron FeNP@FibBLG [triangles] .
   a., b. Typical Michaelis-Menten curves of Fe1@FibBLG [circles], Fe1@BLG [squares]and FeNP@FibBLG [triangles] by varying the ethanol (a) and acetaldehyde (b) concentrations in the presence of H2O2.
   c., d. Comparison of the specific activities (U mg-1) of Fe1@FibBLG, Fe1@BLG and FeNP@FibBLG on ethanol (c) and acetaldehyde (d) oxidation in the presence of H2O2.
   One nanozyme activity unit (U) is defined as the amount of nanozyme that catalyses 1 µmol of product per minute. The specific activities (U mg-1) were determined by plotting the nanozyme activities against their weight and measuring the gradients of the fitting curves. 1H NMR spectrum of reaction products of Fe1@FibBLG catalyzed ethanol (inset c) and acetaldehyde (inset d) oxidation.

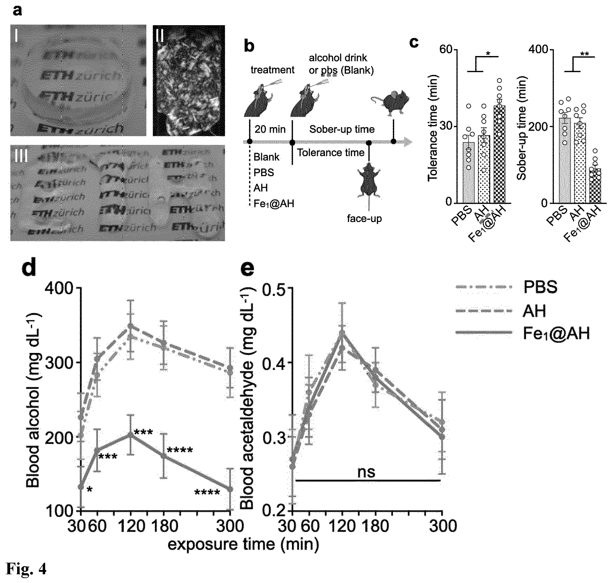

Fig.4 shows the effect of Fe1@AH as a prophylactic for acute alcohol intoxication.
   a, Visualization of Na+-induced Fe1@FibBLG hydrogel (Fe1@AH) (I),
   microstructures of Fe1@AH under polarized light (II) and injectability test (III). b, Schematic of acute alcohol intoxication model construction.
   c, Effect of different treatment (PBS, AH and Fe1@AH) on alcohol tolerance time and sober-up time in C57BL/6 mice.
   d. e. Mean concentrations of blood alcohol (d) and acetaldehyde (e) in alcohol-intoxicated mice treated with PBS, AH and Fe1@AH.
   Data are shown in the form of mean ± SD, n = 8. p values were tested by One-way ANOVA followed by Tukey-Kramer test. * p<0.05, ** p<0.01, *** p<0.001, **** p<0.0001.

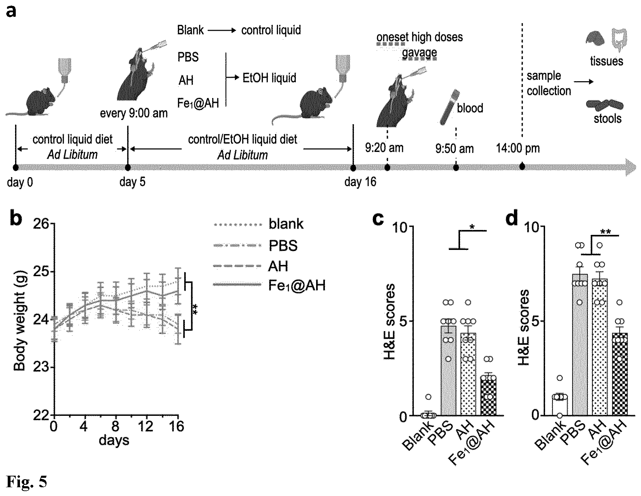

Fig.5 chronic alcohol intoxication
   a. Schematic of the chronic alcohol intoxication model construction.
   b. Body weight changes of 4 groups of mice during feeding period.
   c. Histopathological assessments of liver according to H&E staining in different groups of mice.
   d. Histopathological assessments of colon according to H&E staining in different groups of mice. Data are shown in the form of mean ± SD, n = 8.
   For histopathological and physiological index (b, c, and d), p values were tested by One-way ANOVA followed by Tukey-Kramer test, * p<0.05, ** p<0.01, *** p<0.001.

### Detailed description of the Invention

In more general terms, in a **first aspect,** the invention provides for novel materials containing (i.e. comprising or consisting of) (a) amyloid proteins (preferably amyloid fibrils as described below); (b) iron (preferably in the form of single site iron as described below). These novel materials may be present in the form of hybrid materials as defined herein, or as hydrogels as defined herein.

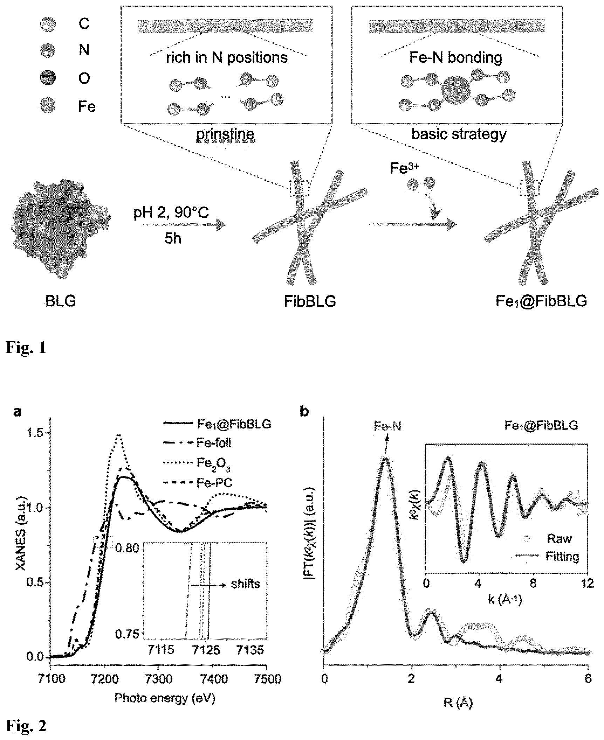

**Hybrid Material:** According to the invention constituents (a) and (b) are in intimate contact. The individual constituents remain separate and distinct within the finished structure but are thoroughly and randomly mixed. This is ensured by the manufacturing process as defined below. The material exhibits properties of both, amyloid fibrils and single site iron, and is therefore termed hybrid material. A schematic presentation is given in fig. 1, showing amyloid proteins (left, without iron), amyloid fibrils (middle, pristine, i.e. without iron), and hybrid material containing amyloid fibrils (a) and single site iron (b) (right).

Advantageously, the ratio (b) / (a) is in the range of 0.2/100 to 7/100 preferably in the range of 0.5/100 to 5/100 (w/w, dry basis as measured by ICP-MS).

**Amyloid fibrils:** The terms "amyloid fibrils" (or "fibrous amyloid protein") is generally known in the field and particularly describes fibrils made by proteins or peptides prevalently found in beta-sheet secondary structure. Accordingly, the term amyloid fibrils excludes native proteins and other types of non-amyloid aggregates.

Amyloid fibrils can be obtained from several different protein sources, such as plant-based proteins and animal-based proteins. Suitable protein sources are referred to as amylodogenic proteins.

In embodiments, amyloid fibrils are obtained from the group of plant-based proteins, preferably:
- zein (e.g. obtained from corn),
- rice protein (e.g. obtained from rice bran),
- cruciferin, and napin (obtained e.g. from rapeseed),
- helianthinin (e.g. obtained from sunflower meal), and
- glycinin, and β-conglycinin (e.g. obtained from soy curd / okara), and

In embodiments, amyloid fibrils are obtained from the group of animal-based proteins, preferably:
- beta-lactoglobulin (FibBLG, e.g. obtained from whey),
- keratin (e.g. obtained from feather),
- lysozyme (e.g. obtained from egg white), and
- BSA (e.g. obtained from whey).

Without being bound to theory, it is believed that a broad range of functionalities characterizes the fibrils obtained from these proteins and, thanks to their remarkable properties, specifically the high number of nitrogen atoms being available for coordination, they have been found particularly suitable for preparing the inventive materials described herein. The amyloid fibrils stabilize atomically dispersed iron and thus function as a nanocarrier.

As an example, the amyloid fibrils are obtained from the group of animal-based proteins, such as whey. Whey is a rich source of proteins whose the most abundant is beta-lactoglobulin, a globular protein that can easily self-assembly into amyloid fibrils. In preferred embodiments, the amyloid fibrils are beta-lactoglobulin (FibBLG).

Advantageously, the amyloid fibrils have high aspect ratio, typically with ≤ 50 nm in radius and ≥ 0.3 µm in length, preferably ≤ 10 nm in radius and ≥ 0.5 µm, in length, particularly preferably ≤ 5 nm in radius and ≥ 1 µm in length.

Advantageously, the amyloid fibrils have a highly charged surface. The term highly charged surfaces is generally known in the field and particularly describes surfaces showing electrophoretic mobilities of the order 2 µm·cm/V·s at pH 4. Electrophoretic mobilities may be determined according to zeta-potential measurement; i.e. colloidal drift mobility under an electronic field.

**Single Site Iron:** The inventive hybrid material comprises a multitude of single-site iron coordinated to said amyloid proteins.

The coordination environment and oxidation state of Fe was investigated using amyloid fibrils of beta-lactoglobulin (FibBLG), the corresponding hybrid material being termed Fe₁@FibBLG. Fei@FibBLG was investigated using X-ray absorption fine structure (XAFS) spectroscopy. The Fe-edge X-ray absorption near-edge structure (XANES) spectra indicated that the pre-edge position for Fei@FibBLG resided between those of Fe foil (metallic iron) and Fe₂O₃, while the white line area located at higher binding energy, demonstrating lower oxidation state and different coordination environments than Fe₂O₃. XANES features are valuable for discerning site symmetry around iron (Fe) in macromolecular complexes. A distinct prominent pre-edge feature before 7120 eV indicates ferrous iron (Fe²⁺) square planar coordination in FePc, while Fei@FibBLG shows a slightly reduced feature due to deviations from perfect square planarity. Notably, the XANES spectra of Fe1@FibBLG (Fig. 2a, inset) closely aligned with that of iron (II) phthalocyanine (FePc). These findings suggest that the Fe within Fei@FibBLG existed in a positively-charged ionic state (Fe6+, where the average δ is close to 2). Further insights were obtained from Fourier transformed (FT) extended X-ray absorption fine structure (EXAFS) spectra in R space (Fig. 2b), which revealed a single peak at approximately 1.4 Å. This peak, based on reference materials, was attributable to the backscattering between Fe and lighter atoms, primarily nitrogen (N) (Fig. 2b). This observation strongly supported the atomic dispersion of Fe sites within Fe₁@FibBLG, corroborating the results from HAADF-STEM analyses. Further, wavelet transform (WT) analysis was conducted, a tool that provides both radial distance and k-space resolution, to discern differences between Fei@FibBLG and reference materials. Unlike the Fe foil and Fe₂O₃ references, WT analysis of Fei@FibBLG revealed a single maximum intensity at approximately 4 Å-1 and 1.4 Å, which was likely due to Fe-N contributions. Fitting results suggested the coordination number (CN) of Fe-N to be 4.5.

Further analysis demonstrates that BLG fibrils possessed effective binding sites that were capable of capturing Fe atoms through Fe-N coordination, enabling the formation of active Fe centers of Fe1@FibBLG.

X-ray photoelectron spectroscopy (XPS) was utilized to ascertain the binding states of carbon (C), nitrogen (N), and iron (Fe) in Fe1@FibBLG. Analysis of these XPS data revealed that approximately 62% of the single-site Fe in Fei@FibBLG was present in the Fe²⁺ state. The existence of Fe²⁺ was ascribed to the reducing effect of BLG fibrils. Collectively, these XPS results not only validated the presence of Fe species within BLG fibrils, but also quantified the charge states of Fe within the BLG fibril framework.

Collectively, these findings confirmed that Fe in the inventive hybrid material exists as single-site Fe and mainly coordinates with N atoms of the amyloid proteins. The findings further confirm that Fe iron is present as Fe⁺² and/or Fe⁺³. It is further believed that the Fe is devoid of any crystalline or oxide Fe metal structure.

**Hydrogel:** The above hybrid material may be converted to an amyloid hydrogel ("Fei@AH"). It was found that the hydrogel retains its biological activity and at the same time provides a convenient oral dosage form. Typical concentration of the hybrid material within the hydrogel is 0.1 - 8 wt.%, preferably 0.5 - 5 wt.%.

Hydrogels are known per se. Typically, a hydrogel is a network of crosslinked polymer chains that are hydrophilic and can thus bind aqueous fluids. They are mainly identified by the monomeric units forming the polymer that in turn can form a hydrogel upon contact with a fluid. Typically, the above fluid is an aqueous fluid, e.g. water and the above polymer are amylodogenic proteins.

The inventive Fe₁@AH showed typical self-standing ability, obvious nanofibril structures evidenced by exceptional birefringence under polarized light. Further, good syringe ability was observed. Fe₁@AH was labelled with ¹⁸F-fluoro-2-deoxyglucose (¹⁸F-FDG) and its transportation was visualized in C57BL/6 mice by using microPET-CT scanning. Metabolism of hydrogel formed Fe₁@AH lasted more than 6 h in the upper GI after gavage, which indicated an extended retention time in vivo which is attributed to the hydrogel nature (Fig. 4b).

In a **second aspect,** the invention relates to pharmaceutical compositions containing the novel materials as described herein (first aspect) and to methods of using such compositions as pharmaceutical, particularly in the context of alcohol detoxification. The skilled person will appreciate that the compositions and methods of using such compositions as described herein provide significant advantages over the prior art.

The present invention provides for a new approach of alcohol detoxification by employing hybrid material, preferably in the form of a hydrogel, that is considered a biomimetic-nanozyme, as an orally-applied catalytic platform. Without being bound to theory, it is believed the amyloid proteins are rich in coordinable N atoms, and thus act as a nanocarrier to stabilize atomically-dispersed Fe. The resulting single-site Fe nanozyme enables efficient catalysis of alcohol oxidation, with a notable advantage of producing less-toxic acetic acid. Administrating the hydrogel as described herein to mice affected by alcoholism, significantly reduced their blood-alcohol levels, without causing additional acetaldehyde build-up, outperforming existing therapies based on natural enzymes. Furthermore, the inventive hydrogel demonstrated a protective effect on the liver, while simultaneously mitigating intestinal damage and dysbiosis associated with chronic alcohol consumption, paving the way for a promising new avenue in effective alcohol detoxification strategies, including prophylaxis treatments. Accordingly, the invention provides for a pharmaceutical composition comprising the hybrid material or the hydrogel as defined herein (first aspect of the invention). In summary, the pharmaceutical compositions described herein are simple in manufacturing, safe in use and effective in the treatment of alcoholism, particularly effective in the precaution / prophylaxis of alcoholism. Consequently, the invention also relates to
- hybrid material and hydrogels as described herein (1^{st} aspect) for use as pharmaceutical;
- hybrid material and hydrogels as described herein (1^{st} aspect) for the treatment of alcoholism;
- the use of hybrid material and of hydrogels as described herein (1st aspect) for manufacturing a medicament for the treatment of alcoholism;
- a pharmaceutical composition comprising an effective amount of a hybrid material or a hydrogel as described herein (1^{st} aspect) for the prevention, treatment or delay of progression of alcohol intoxication ("alcoholism") in a subject, specifically a human being.

The pharmaceutical composition may be administered in any suitable dosage form. It is considered a significant advantage, that the dosage form may be an oral dosage form, such as a capsule, dragée, syrup or the like, each comprising the inventive hybrid material or the inventive hydrogel as described herein.

The pharmaceutical composition may be used for the prevention, treatment or delay of progression of alcohol intoxication ("alcoholism"). In embodiments, the pharmaceutical composition may be used to treat acute alcohol intoxication. In embodiments, the pharmaceutical composition may be used to treat chronic alcohol intoxication.

**Prophylaxis:** To assess whether oral administration of Fe1@AH bestowed any prophylactic benefits in an intoxicated animal model, mice were orally treated with PBS, AH, or inventive Fe1@AH (0.01 mL/g body weight) 30 min prior to one dosage of binge ethanol gavage (12 mg g-1 body weight; n = 8 per group) (Fig. 4c). A group of ethanol-free, but PBS gavaged, mice were set as a negative control (Blank; n = 8), and all the ethanol gavaged mice were asleep for intoxication. Although they struggled for a longer period of time to awaken (approximately 40 min), the Fe1@AH mice were awoken significantly earlier (approximately 2 h) than other intoxicated groups (Fig. 4d). We then conducted the Morris water maze (MWM) test to quantitatively assess murine spatial reference memory 6 h after alcohol consumption (Fig. 4e). Grouped mean swimming speeds of alcohol-exposed mice were comparable to the Blank group, indicating their recovered fundamental activities. However, compared to the mice with a non-alcohol diet, PBS- and AH-treated mice required more time and travelled a longer distance to locate the hidden platform; whereas, the administration of inventive Fe1@AH exhibited a significant reduction in both distance and distance and time (Figs. 4f and 4g). Additionally, the `edge type' and `random type' were predominant searching strategies (37.5%) in PBS and AH groups, while mice that belonged to the Fe1@AH and Blank groups preferred the 'straight line type' and 'tendency type'.

**Chronic alcohol intoxication:** To confirm the long-term beneficial effects of the inventive hydrogel ("Fe₁@AH"), we conducted the NIAAA model (mouse model of chronic and binge ethanol feeding), according to the protocol proposed by Bertola et al.(Nat Protoc 8, 627-637 (2013)) and as further outlined in the examples. This model involved oral administration of Fe1@AH to mice. The model was induced successful, which was characterized by a significantly decreased body weight, increased liver injury (ballooning degeneration and multifocal inflammatory cell infiltration), and hepatic lipid accumulation in mice between Blank and PBS groups (Figs. 5b and 5c). Notably, Fe₁@AH-rescued mice showed a significantly decreased loss in body weight, less liver damage, and re-regulated hepatic lipid metabolism (Figs. 5b and 5c) from intoxication. Moreover, mice treated with Fe₁@AH had lower BA than those with PBS and AH.

In a **third aspect,** the invention relates to methods of manufacturing hybrid materials as described herein (first aspect) and compositions as described herein (second aspect).

**Manufacturing of hybrid material:** The inventive hybrid material is simple in manufacturing; large-scale production is promising using starting materials readily available. Generally speaking, the manufacturing involves a wetness impregnation procedure (Fig. 1a), which includes exposing a dispersion of fibrous amyloid protein in a mixture of polar organic solvent (e.g. ethanol) and dispersant (e.g. PEG200) to an Fe³⁺ solution (e.g. Fe(NO₃)₃) containing dispersant (e.g. PEG200). After multiple rounds of centrifugation and washing, the resulting precipitate was lyophilized to facilitate the secure immobilization of Fe atoms within the fibril structure. Following its synthesis, the hybrid material was subjected to comprehensive characterization and testing. The morphology of the material, which maintained a nanometric diameter consistent with that of pure amyloid fibrils, shows that the integration of Fe did not impact the structural characteristics of the amyloid fibrils. High-angle annular dark-field scanning transmission electron microscopy (HAADF-STEM) and energy-dispersive X-ray spectroscopy (EDS) mapping analysis revealed a significant overlap of the Fe-K profile with the elemental composition of the amyloid fibrils, showing the homogeneous dispersion of the Fe element across the amyloid fibril framework. Atomic force microscopy (AFM) was used to ascertain the height of the fibrils. The findings indicated a consistent height before and after Fe integration, confirming the absence of crystalline Fe or oxide species, within the detection limits of the analytical methods used.

In embodiments, the manufacturing of hybrid material comprises the following steps (i), (ii) and (iii).

**Step (i):** The synthesis of amyloid proteins and of fibrous amyloid proteins is known. Suitable starting materials are food-grade proteins, which are structural stable, widely accessible and inexpensive. Such starting materials allow preparation of amyloid fibrils, such as *β*-lactoglobulin. The self-assembly process is facile and controllable. For fibrils, suitable is in particular protein hydrolysis followed by β-sheets driven fibrillation, as described e.g. in Jung et al. (Biomacromolecules. 2008, 9, 2477-2486). The takes place under acid conditions (e.g. pH2), over a prolonged period of time (e.g. 5 hrs) at elevated temperatures (e.g. 90°C). The thus obtained fibrous material may be further treated.

Typically, lyophilized material is used as a starting material for step (ii).

**Step (ii)** The material of step (i) is dissolved / finely dispersed in a diluent comprising a polar organic solvent (such as an alcohol, e.g. ethanol) and a dispersing agent (such as a polyol, e.g. PEG or ethylene glycol). The obtained first solution is degassed followed by UV irradiation to form free radicals.

A second solution containing a soluble Fe (III) compound, a polar organic solvent and a dispersant is added and allowed to react with said first solution. Suitable Fe+3 compounds are known and commercially available, e.g. iron nitrate.

Reaction typically takes place over prolonged period of time, typically more than one hour, such as 8 - 24 hrs, and moderate temperatures of 20 - 50 °C, typically at room temperature, to thereby obtain the inventive hybrid material in dissolved or finely dispersed form.

**Step (iii)** The hybrid material may be separated from solvents and other components, as known in the field, such as combined centrifugation and washing steps, followed by freeze-drying.

**Manufacturing of hydrogel:** The conversion of amyloid fibrils and of fibrous amyloid proteins into a hydrogel is known per se. Advantageously, the formation of hydrogel is achieved when adding ions, such as sodium ion in the form of NaCl. In a further advantageous embodiment, Au nanoparticles were incorporated as a catalyst for glucose oxidation to produce H2O2.

In embodiments, the manufacturing of inventive hydrogel comprises the following steps (iv), (v) and (vi).

**Step (iv)** providing inventive hybrid material dissolved in aqueous medium. For example, the material obtained from step (iii) may be used. Alternatively, lyophilized hybrid material may be dissolved in aqueous medium. Typically, pure water is used as aqueous medium.

**Step (v)** providing a solution containing finely dispersed gold nanoparticles. Finely dispersed gold nanoparticles, as an auxiliary, is preferably stabilized by amyloid proteins as known in the field. For example, amyloid fibrils are combined with HAUCl₄ solution, the amount of amyloid fibrils: Au precursor typically being > 10 :1. The mixture is subjected to a reduction reaction, such as addition of NaBH4, followed by purification, e.g. dialysis to thereby obtain finely divided Au nanoparticles on the surface of amyloid fibrils.

**Step (vi),** gelation. The materials of steps (iv) and (v) are combined ("fifth solution") and subjected to a gelation reaction. Such gelation reaction is known per se, but not yet applied to the starting materials described herein. Suitably, gelation takes place by slowly combining the fifth solution with acidic saline solution over a prolonged period of time to obtain a uniform hydrogel. Suitable reaction times are at least 1 hour, preferably at least 12 hrs. Suitable conditions include pH=7.4. Suitable low temperatures include 0 - 10°C, such as 4°C. Contacting fifth solution and acidic saline solution may be performed via contacting them via membrane.

To further illustrate the invention, the following **examples** are provided. These examples are provided with no intend to limit the scope of the invention.

### I. Hybrid Material

### Starting materials

(β-lactoglobulin (BLG; >98%) was purchased Davisco Foods International, Inc. and purified.

### Synthesis of inventive hybrid material Fe1@FibBLG:

For the synthesis of Fe1@FibBLG, 100 mg lyophilized BLG fibril powder was dispersed in a mixture of 8.0 mL ethanol and 1.9 mL polyethylene glycol 200 (PEG 200). The dispersion was then subjected to argon bubbling for 30 min to remove the dissolved oxygen, followed by irradiation under a xenon lamp with a UV filter (250 - 380 nm, 27.9 mW cm-2, PLS-SXE300CUV) for 10 min to generate free radicals. Subsequently, 0.1 mL of 108.21 mg mL-1 Fe(NO3)3·9H2O PEG 200 solution was added dropwise to the dispersion of BLG fibrils under magnetic stirring for 12 h at 25 °C.

### Synthesis of inventive hybrid material Fe1@BLG:

Fe1@BLG was prepared with the same synthesis procedure as for Fe1@FibBLG, except that the BLG fibril powder was replaced by an equal amount of BLG powder.

### Synthesis of comparative FeNP@FibBLG

For the synthesis of FeNP@FibBLG, the as-obtained Fe1@FibBLG dispersion was further UV-irradiated for 18 min under anaerobic conditions to reduce the Fe ions.

### Workup

samples were collected by centrifugation at 4 °C, 11,100 × g for 10 min, washed by ethanol (10.0 mL× 6), and re-suspended in 5.0 mL deionized water (pH 2). The powdered Fe1@FibBLG, Fe1@BLG, and FeNP@FibBLG were obtained by lyophilization and stored at 4 °C.

### Analytics

Analytical findings are show in fig.2 and discussed above.

### II. Hydrogels

### Starting materias (AuNPs-attached BLGfibril)

For the synthesis of AuNPs, all glassware was cleaned using freshly prepared aqua regia (HCl:HNO3 = 3:1 volume ratio) and then thoroughly rinsed with water. A 2 mL solution of BLG fibrils (2.0 wt%, pH 2) was mixed with a 40 mM HAuCl4 solution to reach a final protein:Au mass ratio of 14.7:1. The mixture underwent a chemical reduction through the dropwise addition of a NaBH4 solution (0.8 mL) under nitrogen atmosphere. The resulting solution was then dialyzed to remove any remaining NaBH4 and concentrated to 2 mL using a dialysis membrane (Spectra/Por^{®}, MWCO 6-8 kDa, Spectrum Laboratories, Inc.) against a 6 wt % polyethylene glycol solution (Mr ~ 35,000, Sigma-Aldrich) at pH 2. The TEM image of AuNPs stabilized by BLG fibrils revealed three-dimensional particles with an average size of 1.32 nm

### Preparation of Fe1@AH using fibrous amyloid protein

For the preparation Fe1@AH, 2 g of Fe1@FibBLG powder, obtained according to the above protocol, was dissolved in 2mL of the above starting material (AuNPs-attached BLG fibril solution). The mixture was then transferred into a plastic syringe, the top part of which had been previously cut. The plastic syringe was covered with a section of a dialysis tube (Spectra/Por^{®}, MWCO 6-8 kDa), and the head of the syringe was positioned in direct contact with an excessive 300 mM NaCl solution at pH 7.4 for a duration of at least 16 h in a 4 °C cold room to facilitate gelation. The resulting hydrogel sample was kept under 4 °C.

The working hydrogel, i.e. the sample that was directly used for oral administration, was freshly prepared by mixing the aforementioned hydrogel with 0.1 mL of a glucose solution (8.0 M) just before further characterization or detoxication use.

### Preparation of Fe1@AH using amyloid protein

A BLG fibril hydrogel was obtained using the same procedure, except that the Fe1@FibBLG was replaced with an equal amount of BLG fibril dispersion.

### III. In vitro toxicity assessment

Cell viability assays. Six-well plate seeded Caco-2 cells (2 mL and 3×105 cells/well) were treated with AH or Fe1@AH after 24 h of resuscitation under the following conditions: DMEM containing 10%(v/v) fetal bovine serum (FBS), nonessential amino acids, and 1% (v/v) penicillin-streptomycin (10,000 U/mL penicillin and 10 mg/mL streptomycin) at 37 °C in 5% CO2. The same amounts (4.0 g/mL, 40 µL) of AH or Fe1@AH were incubated with cells for another 24 h at 37 °C in 5% CO2. Cells treated with PBS were used as a negative control. After exposure, cells were washed three times in cold PBS, centrifuged at 1500 rpm for 5 min, and re-suspended in 500 µL of binding buffer. The cells were then Annexin V-FITC (Invitrogen^{™}) stained, and the cellular fluorescence was detected by using a flow cytometer (FACSCalibur 2, U.S.A.) equipped with a 488 nm excitation wavelength argon ion laser. Green fluorescence and red fluorescence were measured above 530/30 nm (FL1) and 670 nm (FL3), respectively.

### IV Murine models

### General

Male wild type (WT) C57BL/6 mice, 20-25 g and 8-10 weeks old, were purchased from Beijing Vital River Laboratory Animal Technology Co., Ltd. All of the murine experiments in the current study were approved by the Regulations of Beijing Laboratory Animal Management (approval number: AW40803202-5-1) and conducted according to the guidelines set forth in the Institutional Animal Care and Use Committee of China Agricultural University.

### In vivo safety evaluation.

Male C57BL/6 mice (n=6) were hypodermically injected with the hydrogel according to section II to assess their allergenicity and toxicity. Mouse were anesthetized by intramuscular injections of ketamine (85 mg kg-1 BW) and xylazine (10 mg kg-1 BW) on posterior thighs. After removing back hair, mice were subcutaneously injected with 50 µl of AH or Fe1@AH (1 mg/mL) at time point 0 and 24 h, respectively. Mice injected with H2O2 solution or PBS buffer were set as positive and negative controls, respectively. After the skin test, mice were sacrificed, and skin tissue was collected and fixed for further analysis. Skin samples were stained with hematoxylin and eosin (H&E), and the apoptotic cells were detected by terminal deoxynucleotidyl transferase mediated dUTP nick end labeling (TUNEL, Roche Molecular Biochemicals). Images were captured by Zeiss Axio Vert AI or Zeiss LSM980 (ZEN 2012) confocal microscopes, respectively, and processed with Case Viewer 3.3 software.

### Acute model.

32 male C57BL/6 mice were randomly divided into four groups after 12 h fasting. Mice were orally gavaged with AH and Fe1@AH (at doses of 10 mL kg-1 BW), and two groups of mice received the same volume of PBS (as controls, the Blank and PBS groups), respectively. After 20 min of adaptation, mice from AH, Fe1@AH, and PBS groups were orally administered an alcohol liquid diet (10g kg-1 BW), while the same volume of PBS was administered for the Blank group. All the mice were euthanized 6 h later.

### Chronic model.

A mouse model of chronic and binge ethanol feeding (NIAAA model) was conducted following the protocol proposed by Bertola et al. 38. In brief, after 5 d ad libitum Lieber-DeCarli diet adaptation, 32 mice were randomly divided into four groups: (1) a control group (Con) of mice were pair-fed with the control diet; (2) an ethanol diet group (EtOH); (3) an ethanol diet group with additional 10 mL kg-1 AH; and (4) an ethanol diet group with additional 10mL kg-1 Fe1@AH. The ethanol-fed groups were granted unrestricted access to the ethanol Lieber-DeCarli diet containing 5% (vol/vol) ethanol for 10 d, and additionally received daily morning (9:00 AM) gavage of PBS, AH, or Fe1@AH, respectively. The control group was pair-fed with an isocaloric control diet and daily control-liquid gavage. At day 16, both the ethanol-fed and pair-fed mice were orally administered a single dose of ethanol (5g kg-1 BW) or isocaloric maltose dextrin at 9:30 AM, respectively, and euthanized 6 h later. The body weight of mice was recorded every 2 d.

### MicroPET-CT

After overnight fasting, mice were gavaged with 0.1 mL F-fluorodeoxyglucose (18F-FDG) labeled Fe1@AH. Then, mice were anaesthetized with oxygen containing 2% isoflurane, and placed in and fixed with a prone position in an imaging chamber. Time-series images were obtained by using an Inveon MicroPET/CT scanner (Siemens) and scanner parameters were 15-min CT scan (80 kVp, 500 µA, 1100 ms exposure time) followed by a 10-min PET acquisition. Quantification of images was performed by AMIDE software30.

### Alcohol tolerance test

Approximately 10 µl of blood was collected from the submandibular vein at 30, 60, 90, 120, 180, and 300 min after alcohol exposure. In the chronic model, sampling was conducted after the binge ethanol feeding. Blood alcohol concentration (BAC) was determined using the test kit from Abcam Biotechnology (ab65343). BACs were normalized to mice bodyweights as previously described8. Normalized BAC, BACnor, was calculated using the equation: BACnor = BACi × (BWTi/BWTave), where BACi and BWTi denote the blood alcohol level and bodyweight of mice, respectively, and BWTave represents the average weight of all mice in each set of experiments. The quantification of the blood acetaldehyde concentration (BAceC) was carried out using a test kit obtained from Abcam Biotechnology (ab308327), and the normalization process was conducted using the same method employed for BAC.

Alcohol tolerance time was the duration between alcohol administration and the absence of righting reflex, while the duration of the absence of righting reflex was recorded as sober-up time. Mice becoming ataxic was considered as loss of the righting reflex, and then the mice were placed face-up. The time point at which the mice returned to their normal upright position signified the regaining of their righting reflex.

### Morris water maze (MWM) test

A MWM test was conducted. Specifically, the MWM apparatus contained a large circular pool (120 cm diameter and 40 cm height) which was filled with TiO2 dyed 25°C thermostatic water, and a 10 cm diameter platform was positioned and fixed 2 cm below the water surface. Before acute ethanol exposure, mice received four rounds of daily training for 6 d. Each trial was limited to 60 s, and the time that it took for the mice to successfully locate the platform was recorded. On day 7, mice were re-tested (no platform condition) 5 h after ethanol-feeding (the time point at which all mice restored their consciousness and mobility) The tested item included trajectory, path length, escape latency, and swimming speed (MWM animal behavior video tracking system, Morris V2.0).

## Claims

1. A hybrid material comprising (a) amyloid proteins and (b) a multitude of single-site iron coordinated to said amyloid proteins.

2. The hybrid material according to claim 1, wherein said amyloid protein is a fibrous amyloid protein.

3. The hybrid material according to claim 1 or 2, **characterized in that** said amyloid protein (a) is
▪ a plant-based protein, preferably zein, rice protein, cruciferin and napin, helianthinin, glycinin; or
▪ an animal-based protein, preferably BLG, lysozyme, ovalbumin, serum proteins.

4. The hybrid material according to any of the preceding claims, **characterized in that** said single site iron (b)
▪ is present as Fe⁺² and/or Fe⁺³, and
▪ is coordinated to Nitrogen and/or Oxygen atoms present in said amyloid proteins (a).

5. The hybrid material according to any of the preceding claims, **characterized in that**
▪ the ratio (b) / (a) is in the range of 0.2/100 to 7/100 (w/w, dry basis as measured by ICP-MS); and/or
▪ said amyloid protein (a) is present as fibrous amyloid protein with fibrils being ≤ 50 nm in radius and ≥ 0.3µm in length; and / or
▪ said amyloid proteins (a) show electrophoretic mobilities of the order 2 µm·cm/V·s at pH 4 (as determined according to zeta-potential measurement).

6. A hydrogel comprising the hybrid material as defined in any of the preceding claims.

7. A pharmaceutical composition comprising the hybrid material according to any of claims 1 - 5 or the hydrogel of claim 6.

8. An oral dosage form comprising the pharmaceutical composition according to claim 7.

9. A hybrid material according to any of claims 1-5, or a hydrogel according to claim 6, for use as a pharmaceutical.

10. A pharmaceutical composition according to claim 7, or an oral dosage form according to claim 8,
▪ for the prevention, treatment or delay of progression of alcohol intoxication ("alcoholism");
▪ for the treatment of acute alcohol intoxication; or
▪ for the treatment or amelioration of effects of chronic alcohol intoxication.

11. A method for manufacturing a hybrid material according to claim 1 - 5 comprising the step of exposing a dispersion of (fibrous) amyloid protein in a mixture of polar organic solvent and dispersant ("first solution") to an Fe³⁺ solution containing dispersant ("second solution").

12. The method for manufacturing a hybrid material according to claim 11, comprising the steps of:
step (i) providing a powder of amyloid protein or of fibrous amyloid protein;
step (ii) preparing a first solution by dissolving the powder of step (i) in a diluent comprising polar organic solvent and a dispersing agent, followed by degassing and by UV irradiation; and
combining said first solution with a second solution, said second solution containing a soluble Fe+3 compound, a polar organic solvent and a dispersing agent,
allowing the thus obtained mixture to react, preferably for at least one hour at room temperature, to thereby obtain the hybrid material in dissolved form;
step (iii) optionally separating the hybrid material from solvents and other components, preferably by freeze-drying;
to thereby obtain the hybrid material.

13. A method for manufacturing a hydrogel according to claim 6, comprising the step of exposing a solution comprising hybrid material according to any of claims 1 - 5 and finely dispersed Au nanoparticles ("fifth solution") to an acidic saline solution to thereby allow gelation.

14. The method for manufacturing a hydrogel according to claim 13 comprising the steps of:
step (iv) providing hybrid material according to claim 1-5 dissolved in aqueous medium ("third solution"); and
step (v) providing a solution containing gold nanoparticles, preferably gold nanoparticles finely dispersed on amyloid fibrils ("forth solution"); and
step (vi) combining said third and fourth solution to obtain a fifth solution, and allowing gelation of said fifth solution to take place by slowly combining it with acidic saline solution over a prolonged period of time and low temperatures,
to thereby obtain a uniform hydrogel.
